# EUROPEAN PATENT SPECIFICATION

(11) **EP 1 855 737 B1**
(45) Date of publication and mention of the grant of the patent: **30.12.2009**
(21) Application number: 06737105.4
(22) Date of filing: 03.03.2006
(51) Int. Cl.: A61M 5/14, A61M 5/172

(54) **SYSTEM AND METHOD FOR CONTROLLING ACCESS TO FEATURES OF A MEDICAL INSTRUMENT**
SYSTEM UND VERFAHREN ZUR STEUERUNG DES ZUGRIFFS AUF FUNKTIONEN EINES MEDIZINISCHEN INSTRUMENTS
SYSTEME ET PROCEDE DESTINES A CONTROLER L'ACCES A DES FONCTIONS D'UN INSTRUMENT MEDICAL

(30) Priority: 09.03.2005 US 77370
(43) Date of publication of application: 21.11.2007
(73) Proprietor: Cardinal Health 303, Inc., San Diego, CA 92130 (US)
(72) Inventor: BATCH, Richard, M., Del Mar, California 92014 (US)
(74) Representative: Lampe, Sigmar
(86) International application number: PCT/US2006/007886
(87) International publication number: WO 2006/098927

(56) References cited:
- US-A- 5 658 133
- US-A- 5 713 856
- US-A- 6 039 251
- US-A1- 2002 077 852
- US-A1- 2002 091 454
- US-B1- 6 269 340

## Description

### BACKGROUND

The invention relates generally to a system and method for controlling the configuration of a medical instrument, and more particularly, to a system and method for controlling access to a plurality of features and combinations of features of a medical instrument.

Currently in the medical field, numerous medical instruments are used to provide an array of diagnostic, therapeutic, and patient monitoring capabilities. Many of these devices have a basic set of operating features that place the medical instrument in a basic operating configuration in which the operator has certain basic operating controls over the instrument. In some cases, the medical instrument has or can be supplemented with additional operating features that provide enhanced operating capabilities. However not every clinician or operator of a medical instrument requires or even desires access to all configurations that may be available. Indeed, in the case of a medical instrument that may have the capability to operate in many different configurations or have many different operating features that can be made available, clinicians frequently differ on the configurations they desire to have and reasons for restricting the number of possible configurations. Cost may be a consideration in the choice of operating configurations as instruments with more available operating configurations typically cost more than instruments having fewer operating configurations.

As an example, infusion pumps have advanced significantly over the years and today offer better performance in pumping fluid to the patient. Along with the better pumping performance that is available in even the pump's basic configuration, some infusion pumps also offer a wide range of operating configurations in addition to a basic operating configuration. A large volume parenteral infusion pump ("LVP") typically provides a basic operating configuration with control over basic pumping features, such as the pumping parameters of infusion rate, infusion time, and volume to be infused ("VTBI"). However, additional operating features that may be available or may be made available are multi-dose control, delayed start, bolus dosage control, and drug libraries, as well as others.

With even more complex pumps, whole "practice package" configurations of features may be available. For example, an LVP may have an operating room ("OR") configuration, an oncology ("ONC") configuration, a pediatric ("PED") configuration, a neonatal ("NEO") configuration as well as others. These practice package configurations typically provide settings over a group of features. For example, such a package may include not only predetermined operating parameter limits of the pumping feature but also alarm thresholds, overrides that may be available, and other operating parameters.

While some clinicians may desire to have all of the possible configurations of a pump available, many clinicians desire to have only a select few configurations. In addition to the potential cost savings, limiting the number of features available may simplify the operation of the pump for the clinician and reduce the number of possible pump programming errors that could be made. For example, a healthcare facility may not want a pump to be available in a neonatal ward when that pump has both a NEO configuration and an OR configuration that can be selected. Rather, the clinic may desire that pumps that have only a NEO configuration be available in the ward so that an OR configuration cannot be mistakenly selected. The objective in this case would be to reduce the possibility that an operator would mistakenly program the pump into an OR configuration with its higher pumping parameters and higher alarm limits that may not be suitable for a neonatal patient.

In a present manufacturing process, a pump is manufactured with the basic features necessary to provide the pump's basic operating configuration. Basic pumping features for general use, such as rate, time, and VTBI, are usually available, as described above, in this basic operating configuration. Should the customer order additional features or an additional configuration or configurations for this pump, the manufacturer installs these features during manufacture and tests the pump to validate that each of the installed features operates correctly by itself and operates correctly with all other possible combinations of available features. Thus, the particular combination of features can vary by customer and, if a plurality of features are installed during manufacture, there may also be various combinations of features that can be selected by customers themselves from a control panel of the pump during use. In many cases, such features are controlled by a computer program resident in the pump. The resident computer program typically includes subprograms, each of which controls a feature or combination of features to form a configuration such as a practice package. The additional programming instructions for the additional features or configurations must operate effectively with the basic programming instructions, and must also operate effectively with the programming instructions of each other and with all other program features that are installed in the instrument. All programs must be validated both separately and in combination with each other in all possible combinations. In such a manufacturing approach, the programs in pumps having different installed features must each be validated through separate validation testing phases. This can result in increased time to manufacture a pump as well as higher costs.

US 6 269 340 discloses a system for creating a customised drug library for an electronically loadable drug infusion pump together with a user interface for loading the library.

When an upgrade, correction, or any alteration is made to the resident control program or to one of the separate subprograms that provides certain features, re-validation of it and all combinations of it with the other programs with which it may be combined must be performed. Such intense validation procedures of programming instructions can be a difficult, expensive, and time consuming task. It would be helpful if such extensive validation requirements for computer programming instructions were alleviated.

Hence, those skilled in the art have recognized a need for a system and method that alleviate the burdens of separate validation of various operating features and various combinations and configurations of features in medical instruments. There has also been recognized a need to alleviate the burden of re-validating all features and combinations when a change is made to one or more features. The present invention fulfills these needs and others.

### SUMMARY OF THE INVENTION

Briefly and in general terms, the present invention provides a new and improved system and method for controlling access to features of a medical instrument. The invention is applicable to any multi-featured medical instrument including, but not limited to, infusion pumps and vital signs monitors.

A system for controlling access to operating features of a medical instrument that has a plurality of operating features, the system comprises an input device with which an operator may provide control signals for the operation of the medical instrument, the control signals including selection signals to select single features or combinations of features for operation of the medical instrument, an access key having an access component and a feature control component, and a controller responsive to the access key to determine if the access component is acceptable and if so, to enable and disable particular operating features of the medical instrument in accordance with the feature control component of the access key, wherein the controller is responsive to control signals from the input device to permit operator control over such enabled features and does not permit operator control over such disabled features.

In accordance with further aspects, the controller is responsive to feature packages in which a plurality of features are included in a feature package and if enabled by the access key, permitting the operator of the medical instrument to select enabled feature packages to thereby apply all features in the package to the medical instrument. The controller is also responsive to operator controls from the input device for a basic operating feature regardless of the access device.

In other more detailed aspects in accordance with the invention, the access component comprises an identification of a release number and in another detail; the access component also comprises a program version. The controller and input device form a part of the medical instrument and the controller comprises a program that controls the medical instrument, the program including a plurality of subprograms that control the operating features. The controller is responsive to the feature component of the access key to enable certain subprograms and disable other subprograms. A memory located within the medical instrument wherein the controller is located within the medical instrument and is in communication with the memory, a medical instrument control program is stored in the memory, the control program having a plurality of subprograms that control all operating features of the medical instrument over which an operator could exercise control if the respective subprogram is enabled, and the controller is responsive to the feature component of the access key to enable certain subprograms and disable other subprograms thereby controlling which features of the medical instrument are available for use by an operator.

In still other aspects, the controller presents certain information and certain selectable options for control over features of the medical instrument on a display; however, the controller does not present information about disabled subprograms and features on the display. Enabled features include groups of operating features related to particular locations of use in a medical facility. Further, the controller verifies authenticity of the access key by applying an integrity check.

A method for controlling access to operating features of a medical instrument that has a plurality of operating features, the method comprises providing control signals for the operation of the medical instrument, the control signals including selection signals to select single features or combinations of features for operation of the medical instrument, providing an access signal, the access signal having an access component and a feature control component, determining if the access component is acceptable and if so, enabling and disabling particular operating features of the medical instrument in accordance with the feature control component of the access key, and accepting control signals for operator control over such enabled features and not accepting operator control over such disabled features.

In yet further method aspects, providing an access signal includes providing a feature control component that includes a feature package in which a plurality of operating features are included in a feature package and when the feature package is enabled, accepting control signals for operator control over all features included in the feature package. Providing an access signal includes providing a release number, and includes providing a program version. The method further comprises the step of responding to operator control signals for a basic operating feature regardless of providing an access signal. The method also comprises installing a control program that controls the medical instrument, the control program including a plurality of subprograms that control the operating features and enabling certain subprograms and disabling other subprograms by the control program in response to the feature component of the access key.

In other more detailed aspects of the invention, the method further comprises installing the control program having the plurality of subprograms in a memory located within the medical instrument, the plurality of subprograms controlling all operating features of the medical instrument over which an operator could exercise control if the respective subprogram is enabled and the steps of enabling certain subprograms and disabling other subprograms thereby control which features of the medical instrument are available for use by an operator. The method for controlling access yet further includes displaying certain information and certain selectable options for control over features of the medical instrument and disabling a display of information about disabled subprograms and features on the display. Yet further, the method comprises verifying authenticity of the access signal by applying an integrity check.

In yet further aspects of the invention, there is provided a system for controlling access to subprograms and combinations of subprograms of an application program, the subprograms being operable individually and in selectable combinations with each other, the system comprising an input device with which an operator of the application program may provide control signals for the operation of the application program, the control signals including selection signals to select single subprograms or combinations of subprograms, an access key having an access component and a feature control component, and a controller program responsive to the access key to determine if the access component is acceptable and if so, to enable and disable particular subprograms of the application program in accordance with the feature control component of the access key, wherein the controller program is responsive to control signals from the input device to permit operator control over such enabled subprograms and does not permit operator control over such disabled subprograms.

In more detailed aspects, the controller program is responsive to subprogram packages in which a plurality of subprograms are included in a subprogram package and if enabled by the access key, permitting the operator of the application program to select enabled feature subprograms to thereby operate all subprograms in the package. The access component comprises identification of a release number and in a further aspect; the access component also comprises an identification of a program version. In another aspect, the controller program is responsive to operator controls from the input device for a basic subprogram regardless of the access device.

Other aspects and advantages of the invention will become apparent from the following detailed description, taken in conjunction with the accompanying drawings, which illustrate, by way of example, the features of the invention.

### BRIEF DESCRIPTION OF THE DRAWINGS.

FIGURE 1 is a front view of a medical instrument taking the form of a modular patient care system including a controller, an infusion pump mounted at the left of the controller, a syringe pump module mounted to the right of the controller, and an oximetry module mounted at the right of the syringe pump;
FIG. 2 is a block diagram of components of the controller of FIG. 1;
FIG. 3 is a block diagram of components of the infusion pump module shown in FIG. 1 that forms part of the medical instrument;
FIG. 4 is a block diagram showing an example of a medical instrument having a plurality of operating features available for use individually, and which may be grouped to form operating features configurations, and also showing a feature access key and a controller responsive to the feature access key, in accordance with aspects of the invention;
FIG. 5 is a block diagram of the components of a feature access key according to aspects of the present invention; and
FIG. 6 is a flow chart diagramming a method of selecting operations features of a medical instrument through the use of a feature access key to derive an operating configuration, according to aspects of the present invention.

### DETAILED DESCRIPTION OF PREFERRED EMBODIMENTS

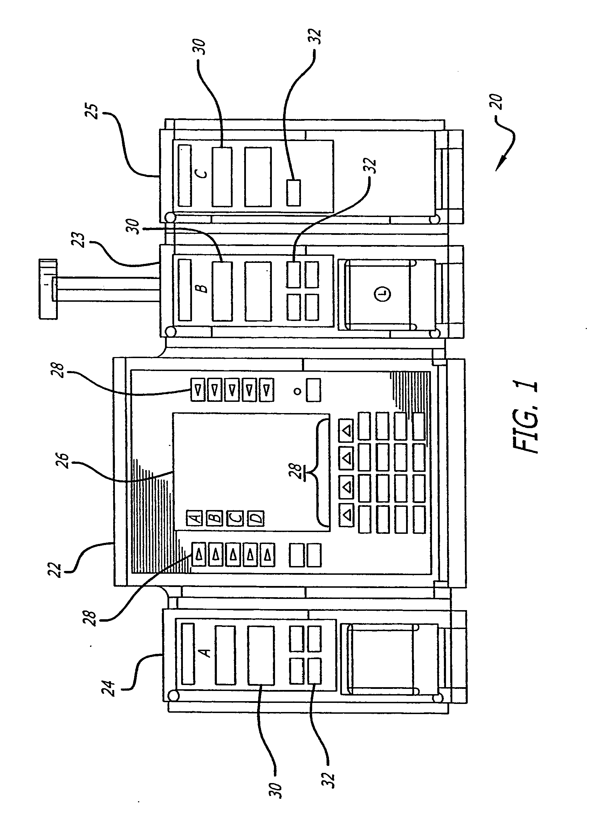

Referring now to the drawings in which like reference numerals refer to like or corresponding elements among the several figures, there is shown in FIG. 1 a front view of a medical instrument 20, which in this embodiment comprises a modular patient care system having a controller 22 located in the center and three functional clinical devices that are mounted on either side of the controller. In this case, the clinical devices comprise an infusion pump module 24 mounted to the left side of the controller, a syringe pump 23 mounted to the right side of the controller, and an oximetry module 25 mounted to the right side of the syringe pump, also to the right of the controller. The infusion pump and syringe pump provide medical fluids to the circulatory system of a patient while the oximetry module monitors the level of oxygen in the blood of a patient. A system of this sort is more fully described in U.S. Patent No. 5,713,856 entitled "Modular Patient Care System" to Eggers et al.. The controller comprises a processor and memory for storing data and programs and for executing those programs. Data may be obtained from the clinical devices 23, 24, and 25 interacting with the controller and the programs are for controlling the operation of the controller and the clinical devices. While the following embodiments of the present invention will be described in the context of a modular patient care system, various types of medical instruments, including those forming individual standalone units, as well as modular systems, are contemplated. Further, when applied to a modular system, the clinical devices interacting with the controller need not be in physical contact with the controller.

The infusion pump module 24 depicted in FIG. 1 is a large volume parenteral pump ("LVP") configured to deliver fluid at a controlled rate to a patient (not shown). In addition to the infusion pump mounted to the controller 22 and controlled separately by the controller, other functional medical instruments or clinical devices or modules, such as those providing patient monitoring, may be linked together with the infusion pump or other instrument in a practice configuration. For example, the syringe pump 23 may be linked with the oximetry medical instrument 25 to operate in a patient-controlled analgesia ("PCA") configuration. In such an arrangement, the syringe pump delivers analgesia to the patient on the command of the patient; however, the controller may override the patient's command based on the data concerning the patient's blood-oxygen saturation provided by the oximetry medical instrument. In such an example, the controller is enabled to provide the features of the infusion pump, the features of the syringe pump, and the features of the oximetry instrument, as well as provide further features in operating the syringe pump and the oximetry instrument in a PCA practice configuration. Other examples of features that may be provided by the controller and instruments or modules mounted with and under the control of the controller include capnography monitors, invasive or non-invasive blood pressure monitors, and others.

In the case of the medical instrument 20 shown in FIG. 1 and in other figures, the clinical devices including the infusion pump 24, the syringe pump 23, and the oximetry module 25 can be connected on their left or right sides to the controller 22 or to another clinical device. In the example shown, the right side of the infusion pump is mounted to the left side of the controller and the left side of the syringe pump is mounted to the right side of the controller. Other clinical or modules, including another infusion pump or pumps, may be mounted to the existing clinical devices. Further details may be found in U.S. Patent No. 5,713,856 to Eggers.

In this embodiment, the controller 22 provides a centralized interface for the various attached functional clinical devices 23, 24, and 25, performing various functions for the clinical devices such as programming and communications. In this embodiment, the controller is also used to provide power to the mounted clinical devices, to provide an interface between the patient care system 20 as a whole (including the attached modules) and external servers, and other devices, and to provide most of the clinician interface for the pumps and the oximetry module. The controller includes a display 26 for visually communicating various information, such as the operating parameters of the pump, as well as displaying alerting and alarm messages. The controller also includes control keys 28 for programming the attached functional clinical devices.

As is further shown in FIG. 1, the infusion pump 24 also has a display 30, such as an LED display, located in plain view on the door in this embodiment and may also be used to visually communicate various information relevant to the pump, such as the current infusion rate and alarm messages. Control keys 32 also located on the front panel of the infusion pump exist for controlling certain operations of the infusion pump, such as pausing or resuming an infusion. Both of the other instruments 23 and 25 mounted to the controller have displays 30 and control keys 32 also.

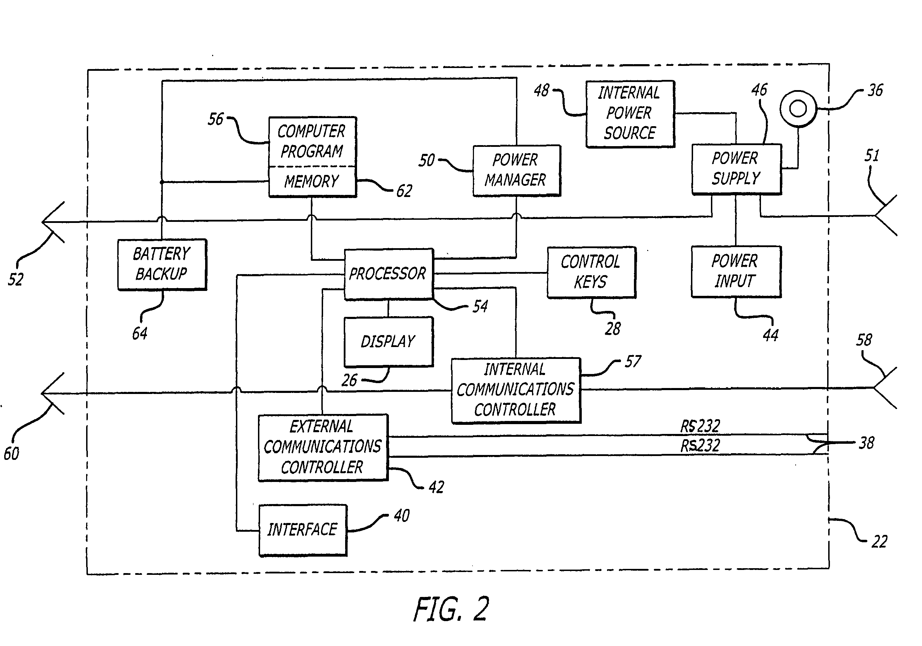

Referring now to FIG. 2, a block diagram of components of the controller 22 is shown. The controller includes an audio alarm 36 that may be used in conjunction with the display 26 to alert the operator to various conditions, such as programming or operating errors, alarms, and other advisories. The controller also has external communication ports 38, such as RS-232 serial ports, with which it may communicate with a medical facility server or other computer and with a portable processor that a clinician may have to transfer information as well as to download drug libraries or other data and programs. In addition, the controller in one embodiment includes a communications interface 40 which provides a personal computer memory card international association (PCMCIA) slot for receiving PCMCIA cards. The examples presented here are for illustration purposes only, and one skilled in the art could readily select from a variety of commercially available communication means. For instance, the controller may be equipped with various wired systems and with various wireless systems, such as an RF (radio frequency) system, an infrared system, a Blue Tooth system, or other. In the embodiment shown in FIG. 2, an external communications controller 42 controls the command and data flow through the ports 38, while the processor 54 of the controller directly controls the communications interface 40. The external communication ports 38, communications interface 40, and control keys 28 may all be used as input devices through which information may be entered to the system 20. The controller may also include other input devices such as a bar code scanner (not shown) for scanning information relating to the infusion or RFID interrogator for reading information from passive or active RFID tags.

The controller 22 contains a power input 44 for receiving power from an external power source (not shown) and forwarding that power to a power supply 46. Additionally, an internal power source 48, such as a battery, may be used to maintain power to the system functions, including memory, when the controller is disconnected from an external power source. A power manager 50 controls the switchover between the two power sources, controls the charging of the internal power source and monitors the remaining capacity and power consumption of the internal power source to estimate the remaining system runtime on the internal power source. The power supply also provides power to any attached modules, such as the infusion pump 24, through power ports 51 and 52.

The processor 54 accesses and executes a computer program or programs 56 that control the operation of the overall medical instrument, i.e. patient care system 20, including aspects of any attached medical instrument modules. The processor 54 communicates with the attached modules via an internal communications controller 57 that controls the command and data flow to the attached modules through the internal communications ports 58 and 60. In this embodiment, the computer program 56 is resident in the controller 22 of the patient care system 20, as shown in FIG. 2. More particularly, the computer program is stored or embedded in a memory 62 in one embodiment. It is to be understood that the memory 62 as well as other memories in the patient care system 20, may be any type of memory or any combination of memories that can be erased and/or reprogrammed without having to physically remove the memory from the system. Examples of such memories include, but are not limited to, battery-backed random access memory (RAM) and "flash" electronically erasable programmable read only memory (FLASH EEPROM). A battery backup 64 provides power to the memory 62 in the event of loss of power from both the external and internal 46 and 48 power supplies.

The program 56 provides control over the controller features and the features of the medical instruments mounted to the controller, in this case the infusion pump 24, the syringe pump 23, and the oximetry instrument 25. An example of controller features may be the basic operating controls over certain mounted instruments such as rate, time, and VTBI for the pumps. Another example may be the acceptable oxygen saturation settings (high and low) for the oximetry module. Additional features for the controller may be certain displays of trends for example, or identification of the patient, medication being administered, or drug libraries. In addition, the program may provide for the ability to program practice packages as described above. The features associated with PCA may be made available by the program. In such a case, the data of the oximetry instrument is analyzed and used to control the syringe pump. Many other features may be made available by the program 56. The program typically has a main control program with a plurality of subprograms directed to a single feature, multiple features, predetermined combinations of features, or practice packages.

Additionally, each medical instrument mounted to the controller may have its own resident program that will likewise provide features of operation of the respective instrument. For example, the program in each pump instrument 23 and 24 may have the features of rate, time, and VTBI while the program in the oximetry instrument may provide the features of the acceptable oxygen saturation settings (high and low).

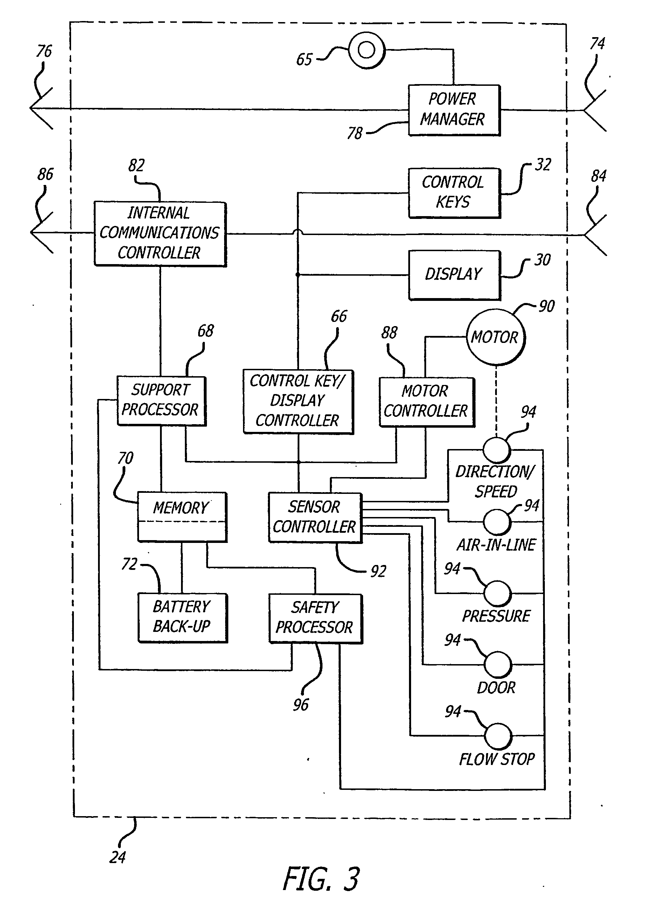

FIG. 3 is a block diagram that illustrates components of the infusion pump medical instrument 24. Along with the display 30, an audio alarm 65 serves to alert the operator to certain conditions, such as the completion of an infusion or a downstream or upstream occlusion in a fluid line for example. The display 30 and control keys 32 are controlled by the control key/display controller 66. The infusion pump 24 receives and processes data and commands from the clinician and communicates with the attached controller 22 through a support processor 68 and an associated memory 70. As in the controller, the memory has a battery backup 72 that assists in retaining stored data and programs during periods where the main power source is not available. The infusion pump receives power from the controller via one or the other of the power ports 74 and 76 depending upon which side of the controller the infusion pump is mounted. A power manager 78 controls the distribution of the power from the power ports 74 and 76 to the pump. The pump also contains an internal communications controller 82, which may send or accept data or commands from the controller through the communication ports 84 and 86, again depending upon which side of the controller 22 the infusion pump is mounted.

The infusion pump 24 also contains typical components of commercially available pumps, such as a motor controller 88 for controlling a pump motor 90 and a sensor controller 92 to obtain indications from sensors 94 which illustratively may be used to detect pump mechanism speed and fluid pressure, air-in-line, and flow stoppage. Other sensors may exist in other embodiments and may be monitored by the support processor 68 or other devices. The indications received by the sensor controller are monitored by the support processor as well as a safety processor 96 to activate alarms and/or stop the operation of the pump when undesired events are detected. The motor controller 88 and pump motor 90 may be any suitable peristaltic pump motor/motor controller combination.

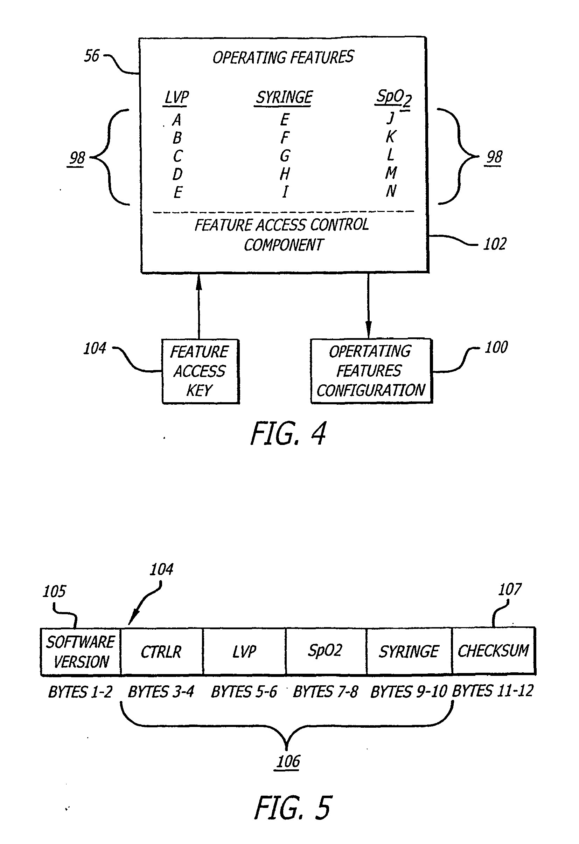

FIG. 4 shows a block diagram generally illustrating an embodiment of the computer program 56 in accordance with principles of the invention. As mentioned above, the computer program is resident in the controller 22 (FIG. 1) in this embodiment and controls the operation of the medical instrument 20. The resident program provides a plurality of operating features 98 that may be combined in various combinations to form different operating configurations 100 for the system 20. Although the program can provide features of the controller itself, the designation "controller" has been left off FIG. 4. Instead, other features related to the pumps and oximetry (SpO₂) instrument are discussed.

The various operating features of the infusion pump 24 ("LVP") of FIGS. 1 and 3 are illustrative of types of operating features 98 the resident computer program 56 may provide a medical instrument. The pump typically provides several basic operating features including flow rate control, control over the infusion time, and control over the volume-to-be-infused. The basic operating features may also include the activation of alarms in response to error conditions detected by the various sensors 94. Features are indicated symbolically through the use of the alphabetical characters "A through E" for the infusion pump, "E through I" for the syringe pump, and "J through N" for the oximetry instrument.

In addition to these basic operating features, the infusion pump 24 or syringe pump 23 may include other operating features. Whether located in the controller 22 or the pump itself, the feature of a drug library that stores data such as drug names, concentrations, rates, and maximum allowable doses, or other parameters may be provided for operation of the infusion or syringe pump. The feature of a drug infusion rate calculator may also be provided. Further, the features of complex drug delivery procedures, such as multiple rate volume infusions and automated ramp up-taper down infusions may be provided. Features of multi-channel coordinated infusions, multi-dose infusions, secondary or "piggyback" infusions, bolus dosage and delayed start infusions may also be made available by the program.

The controller 22, pumps 23 and 24, and oximetry instrument 25, as well as other instruments that may be mounted or connected together, may also support features related to "practice packages." Operating features tailored for particular practice areas or locations of use in a hospital such as the operating room ("OR"), oncology ("ONC"), or pediatrics ("PED") wards or rooms may be made available. These practice packages may provide, for example, the specific operating parameters, alarm thresholds and available overrides appropriate for the designated practice area or hospital location as well as other specific practice-related data, controls, and displays of information.

Although various operating features 98 have been described with respect to the medical instrument configuration shown in FIG. 1 consisting of two pumps 23 and 24 and a patient monitor (oximetry) instrument 25, a plurality of operating features 98 may similarly be provided for other modules that may be mounted to form a part of a patient care system 20. In the embodiment shown in FIG. 4, a pulse oximeter (SpO₂) and syringe pump, each having a plurality of operating features 98, form a part of the patient care system 20 along with the infusion pump. In the case shown and described in FIGS. 1 through 4, the resident program 56 (FIG. 2) is a complete program including all subprograms necessary to operate the controller 22 with any instrument that may be mounted to it directly or indirectly and to provide any and all features that it and such instruments are capable of providing to a clinician or other operator in any combination or practice package. Basic operational features included in the program as well as the most advanced and complex features designed. Therefore, only one program is needed for any such medical instrument because that program contains all features that exist as of the day the program is installed into a medical instrument. However, in accordance with aspects of the invention, access to those features is controlled, as discussed below.

The computer program 56 controls access to the various operating features 98 of the medical instruments with which it is associated. In particular, the operating features 98 may be selectively activated or deactivated to form combinations of operating features to place the instrument in the various operating configurations 100. The computer program 56 includes a feature access control component 102 that inhibits the use of an operational feature unless it has been activated. In one embodiment, certain basic operational features may be available at all times, regardless of the feature access control component status. For example, the basic features of rate, time, and VTBI may always be available to any operator. However, in another embodiment, no operational feature is available without the respective feature being activated by the feature access control component.

In accordance with aspects of the invention, a feature access key 104 may be used with the feature access control component 102 to activate operational features of the medical instrument. The feature access control component will selectively activate and deactivate features depending on the contents of the feature access key 104. The feature access key may be communicated to the processor 54 (FIG. 2) via any of the input devices of the medical instrument, such as the control keys 28 (FIG. 1) or interface 40 such as by use of a personal digital assistant ("PDA") or other device. The key may also be given by remote server in the case where the medical instrument is in contact with it.

In another embodiment, the computer program 56 is responsive to the access key 104 to activate a plurality of different features, configurations, and practice packages. Additionally in one embodiment, an access key exists that will activate all possible features of the medical instrument. Such access key is typically termed a "master" access key and is independent of the release number or version number of the program.

Turning now to FIG. 5, an embodiment of a feature access key 104 according to aspects of the present invention is shown. In this embodiment, the feature access key is a binary string, although it will be understood by one skilled in the art that the access key may be provided in other forms as well, such as an alphanumeric string. In FIG. 5, the feature access key 104 includes twelve bytes, with the first two bytes 105 being used to identify the version number of the computer program. In one embodiment, the version number contains two parts, a release number, which is stored in the first byte, and a revision number, which is stored in the second byte. The version number identified by the access key must match the actual version number of the computer program of the medical instrument for the access key to be valid in this embodiment.

A data area 106 follows the software version number in the binary string. The data area 106 contains a two-byte section for the CTRLR (controller) and each module type supported by the controller, which, in FIG. 5, includes an LVP (infusion pump), an SpO₂ (pulse oximeter), and a SYRINGE (syringe pump). Although only three module types are supported in the embodiment of FIG. 5, the data area 106 may be expanded to support any number of module types that are part of the medical instrument. The first bit in each two-byte section for the modules indicates whether the module is supported by the patient care system. Two bits per byte, such as the lowest bit in each nibble, are reserved for use with an integrity check, or an error checking technique, such as cyclical redundancy checking ("CRC") or, as in this embodiment, a checksum. The remaining bits in each two-byte section indicate which optional operating features associated with the particular module type are to be activated and deactivated.

The last two bytes 107 of the access key 104 in this embodiment are used for a checksum. The checksum provides a security feature against attempts to enter an access key that activates features that have not been authorized for use. The reserved bits in the data area 106 are manipulated to give a desired checksum value that is stored in the last two bytes 107. The form of the feature access key may vary, only one embodiment is shown and described for purposes of illustration. For example, other data integrity checks or data error checking techniques may be used, such as but not limited to a CRC technique or similar data integrity checks.

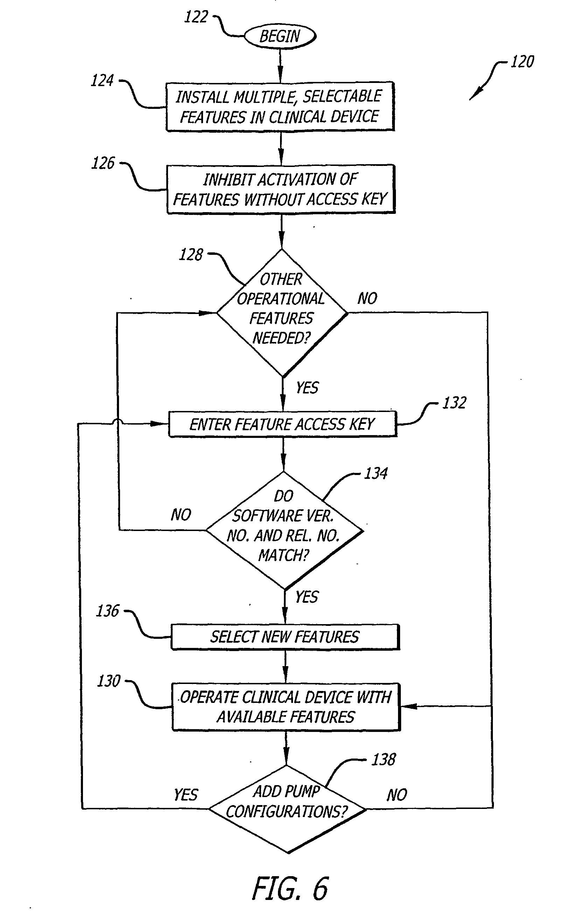

FIG. 6 shows a method 120 for controlling access to features of a medical instrument, according to aspects of the present invention. This particular medical instrument has a plurality of operating features such as shown in FIG. 4 that are combinable in different combinations or usable alone. These features control the operation of the medical instrument. The method is begun at 122 and at step 124, a program that includes all operating features that may be selectively activated or deactivated in combinations to place the medical instrument in any of a plurality of operating configurations is installed. The entire computer program is installed in the memory of a controller of the medical instrument such that the computer program is resident within the system available to the controller and controls the operation of the medical instrument and the selection of the operating configuration of the system. Step 124 may be performed at the manufacturing site for the medical instrument; installation of the computer program may also occur through flash upgrading in the memory to include new features and combinations of features available for possible selection by the controller.

The program inhibits 126 the use or activation of features, combinations of features, and practice packages without an access key. As discussed above, in one embodiment, certain operational features may be available regardless of the existence of an access key and a decision 128 may be made that these are sufficient for the operation of the pump 130 for the present patient. For example, the features of rate, time, and VTBI may be available for use with the pump and this may be sufficient for present purposes. However, if other features are desired 128, the method now requires the entry of an access key 132. The access key is checked 134 and if certain information correlates thereby authenticating the access key, the data of the access key is received and is used to activate operational features of the medical instrument 136. The pump is now operated 130 with the additional features. If the access key cannot be authenticated, the method returns to the decision box of "other operational features needed?" 128.

During operation of the medical instrument, such as the pump example used in FIG. 6, if it is decided that further pump configurations are needed 138, an access key is entered 132 so that those configurations may be activated.

The use of the access key in accordance with aspects of the invention provides for a single program to be validated for all medical instruments instead of multiple programs that have been customized for each customer order. In accordance with the invention, all programs and subprograms necessary to control the medical instrument are resident in the medical instrument and simply need to be activated to be available for use. No further installations of programs are necessary to obtain further features. For example, marketing or sales personnel assisting a customer in the selection and use of the medical instrument features and configurations can identify which operating configurations the customer may find useful and may simply provide the customer with the necessary access key corresponding to that operating configuration or configurations at that time. The customer, or the sales personnel, can then enter the feature access key into the controller of the medical instrument via an appropriate input device to obtain the desired operating configuration. In another embodiment, the access key may be entered into the medical instrument by the manufacturer remotely over the Internet or other communication means so that the customer need not become involved in such reprogramming.

At step 136, the feature access control component 102 (FIG. 4) responds to the feature access key 104 to activate and deactivate respective operating features 98 of the medical instrument. As a result, the instrument may be placed in the particular operating configuration or configurations corresponding to the access key data fields. The clinician may then operate the medical instrument accordingly. In one embodiment, operating features that have not been activated or that have been deactivated by the access key are not displayed as options on the display 26 or shown any other way. Accordingly, the clinician would not be distracted by options that can be seen but not used.

In a further embodiment, attempted use of an incorrect feature access key will result in the medical instrument ignoring the incorrect access key. In another embodiment, the attempted use of an incorrect access key will cause the medical instrument to revert to the basic feature mode where the only features activated are those that do not require the use of an access key.

In the case of one embodiment, once activated, the features are not deactivated unless the customer requests such action. However, at the time of upgrade to the program (including the subprograms), the customer is once again queried as to any changes he/she desires to available features of the medical instrument. If the customer no longer desires certain features, they are simply not activated during upgrade of the program in the medical instrument. Upgrading the computer program can be performed in typical ways, such as through the distribution of any appropriate computer readable medium, such as a PCMCIA card or a CD-ROM, or may be directly installed through connection with the Internet or other data communication means. In the case where a medium is distributed to the customer, a medical instrument technician of the healthcare facility in which the medical instrument is located installs the entire computer program in the memory of the medical instrument. The technician is given an access key accompanying the upgrade medium according to the ordered configurations of the customer and he/she enters the access key to place the instrument in the configuration with the desired features.

Although primarily discussed in terms of an infusion pump, the system and method in accordance with the invention are usable with other medical instruments. An oximetry instrument was also shown but other monitoring and healthcare instruments can incorporate aspects of the invention.

From the foregoing, it will be appreciated that the system and method in accordance with the principles of the invention provide a convenient means to selectively control access to various features in a multi-featured medical instrument to accommodate individual clinicians. A manufacturer can support and validate a single program while still selectively controlling access to features of the computer program. Such selective activation may be provided by various manufacturer personnel who can provide each customer with a specific feature access key that corresponds to a particular operating configuration selected by the customer.

Although specific embodiments of the invention have been described and illustrated, it may be seen that the invention is susceptible to modifications and other embodiments within the ability of those skilled in the art, and without the exercise of the inventive faculty. Thus, it should be understood that various changes in form, detail, and application of the present invention may be made without departing from the scope of the invention.

## Claims

1. A system for controlling access to operating features of a medical instrument that has a plurality of operating features, the system comprising:
an input device with which an operator may provide control signals for the operating of the medical instrument, the control signals including selection signals to select single features or combinations of features for operation of the medical component, an acces key having an access component and a feature control component, wherein the feature control component includes a data area indicating which of the operating features are to be activated or deactivated; and
a controller responsive to the access key to determine if the access component is acceptable and if so, to enable and disable particular operating features of the medical instrument in accordance with the data area of the feature control component of the access key, wherein the controller is responsive to control signals from the input device to permit operator control over such enabled features and does not permit operator control over such disabled features.

2. The system for controlling access of claim 1 wherein the controller is responsive to feature packages in which a plurality of features are included in a feature package and if enabled by the access key, permitting the operator of the medical instrument to select enabled feature packages to thereby apply all features in the package to the medical instrument.

3. The system for controlling access of claim 1 wherein the access component comprises an identification of a release number.

4. The system for controlling access of claim 3 wherein the access component also comprises an identification of a program version.

5. The system for controlling access of claim 3 wherein the controller is responsive to operator controls from the input device for a basic operating feature regardless of the access key.

6. The system for controlling access of claim 1 wherein the controller and input device form a part of the medical instrument.

7. The system for controlling access of claim 1 wherein:
the controller comprises a program that controls the medical instrument, the program including a plurality of subprograms that control the operating features; and
the controller is responsive to the feature component of the access key to enable certain subprograms and disable other subprograms.

8. The system for controlling access of claim 7 further comprising a memory located within the medical instrument wherein:
the controller is located within the medical instrument and is in communication with the memory;
a medical instrument control program is stored in the memory, the control program having a plurality of subprograms that control all operating features of the medical instrument over which an operator could exercise control if the respective subprogram is enabled; and
the controller is responsive to the feature component of the access key to enable certain subprograms and disable other subprograms thereby controlling which features of the medical instrument are available for use by an operator.

9. The system for controlling access of claim 8 further including a display, wherein:
the controller presents certain information and certain selectable options for control over features of the medical instrument on the display; and
the controller does not present information about disabled subprograms and features on the display.

10. The system for controlling access of claim 7 wherein enabled features include groups of operating features related to particular locations of use in a medical facility.

11. The system for controlling access of claim 1 wherein the controller verifies authenticity of the access key by applying an integrity check.

12. A method for controlling access to operating features of a medical instrument that has a plurality of operating features, the method comprising:
providing control signals for the operation of the medical instrument, the control signals including selection signals to select single features or combinations of features for operation of the medical instrument;
providing an access signal, the access signal having an access component and feature control component, wherein the feature control component Includes a data area indicating which of the operating features are to be activated or deactivated;
determining if the access component is acceptable and if so, enabling and disabling particular operating features of the medical instrument in accordance with the data area of the feature control component of the access key; and
accepting control signals for operator control over such enabled features and not accepting operator control over such disabled features.

13. The method for controlling access of claim 12 wherein:
providing an access signal includes providing a feature control component that includes a feature package in which a plurality of operating features are included in a feature package; and
when the feature package is enabled, accepting control signals for operator control over all features included in the feature package.

14. The method for controlling access of claim 12 wherein providing an access signal includes providing a release number.

15. The method for controlling access of claim 12 wherein providing an access signal includes providing a program version.

16. The method for controlling access of claim 12 further comprising the step of responding to operator control signals for a basic operating feature regardless of providing an access signal.

17. The method for controlling access of claim 12 further comprising:
installing a control program that controls the medical instrument, the control program including a plurality of subprograms that control the operating features; and
enabling certain subprograms and disabling other subprograms by the control program in response to the feature component of the access key.

18. The method for controlling access of claim 17 further comprising installing the control program having the plurality of subprograms in a memory located within the medical instrument, the plurality of subprograms controlling all operating features of the medical instrument over which an operator could exercise control if the respective subprogram is enabled; and
the steps of enabling certain subprograms and disabling other subprograms thereby control which features of the medical instrument are available for use by an operator.

19. The method for controlling access of claim 18 further including displaying certain information and certain selectable options for control over features of the medical instrument; and
disabling a display of information about disabled subprograms and features on the display.

20. The method for controlling access of claim 12 further comprising verifying authenticity of the access signal by applying an integrity check.

## Patentansprüche

1. System zur Steuerung des Zugriffs auf Betriebsfunktionen eines medizinischen Instruments, das eine Vielzahl von Betriebsfunktionen hat, wobei das System umfasst:
eine Eingabevorrichtung, mit der ein Bediener Steuersignale zum Betreiben des medizinischen Instruments bereitstellen kann, wobei die Steuersignale Auswahlsignale zum Auswählen einzelner Funktionen oder Kombinationen von Funktionen für den Betrieb der medizinischen Komponente aufweisen, wobei ein Zugriffsschlüssel eine Zugriffskomponente und eine Funktionssteuerungskomponente hat, wobei die Funktionssteuerungskomponente einen Datenbereich aufweist, der angibt, welche der Betriebsfunktionen zu aktivieren oder zu deaktivieren sind; und
eine Steuereinrichtung, die auf den Zugriffsschlüssel anspricht, um zu bestimmen, ob die Zugriffskomponente annehmbar ist, und wenn ja, um bestimmte Betriebsfunktionen des medizinischen Instruments entsprechend dem Datenbereich der Funktionssteuerungskomponente des Zugriffsschlüssels freizugeben und zu sperren, wobei die Steuereinrichtung auf Steuersignale von der Eingabevorrichtung anspricht, um Bedienerkontrolle über solche freigegebenen Funktionen zu erlauben und Bedienerkontrolle über solche gesperrten Funktionen nicht zu erlauben.

2. System zur Steuerung des Zugriffs nach Anspruch 1, wobei die Steuereinrichtung auf Funktionsspakete anspricht, wobei eine Vielzahl von Funktionen in einem Funktionspaket enthalten ist, und wenn durch den Zugriffsschlüssel freigegeben, dem Bediener des medizinischen Instruments erlaubt wird, freigegebene Funktionspakete auszuwählen, um **dadurch** alle Funktionen in dem Paket auf das medizinische Instrument anzuwenden.

3. System zur Steuerung des Zugriffs nach Anspruch 1, wobei die Zugriffskomponente eine Bezeichnung einer Versionsfreigabenummer umfasst.

4. System zur Steuerung des Zugriffs nach Anspruch 3, wobei die Zugriffskomponente auch eine Bezeichnung einer Programmversion umfasst.

5. System zur Steuerung des Zugriffs nach Anspruch 3, wobei die Steuereinrichtung auf Bedienereinstellungen von der Eingabevorrichtung für eine Basisbetriebsfunktion ungeachtet des Zugriffsschlüssels anspricht.

6. System zur Steuerung des Zugriffs nach Anspruch 1, wobei die Steuereinrichtung und Eingabevorrichtung einen Teil des medizinischen Instruments bilden.

7. System zur Steuerung des Zugriffs nach Anspruch 1, wobei:
die Steuereinrichtung ein Programm umfasst, das das medizinische Instrument steuert, wobei das Programm eine Vielzahl von Unterprogrammen aufweist, die die Betriebsfunktionen steuern; und
die Steuereinrichtung auf die Funktionskomponente des Zugriffsschlüssels anspricht, um bestimmte Unterprogramme freizugeben und andere Unterprogramme zu sperren.

8. System zur Steuerung des Zugriffs nach Anspruch 7, ferner umfassend einen Speicher, der sich innerhalb des medizinischen Instruments befindet, wobei:
die Steuereinrichtung sich innerhalb des medizinischen Instruments befindet und mit dem Speicher in Verbindung steht;
ein medizinisches Instrumentensteuerungsprogramm in dem Speicher gespeichert ist, wobei das Steuerungsprogramm eine Vielzahl von Unterprogrammen hat, die alle Betriebsfunktionen des medizinischen Instruments steuern, über die ein Bediener die Kontrolle ausüben kann, wenn das jeweilige Programm freigegeben ist; und
die Steuereinrichtung auf die Funktionskomponente des Zugriffsschlüssels anspricht, um bestimmte Unterprogramme freizugeben und andere Unterprogramme zu sperren, wodurch darüber bestimmt wird, welche Funktionen des medizinischen Instruments zur Verwendung durch einen Bediener verfügbar sind.

9. System zur Steuerung des Zugriffs nach Anspruch 8, ferner mit einer Anzeige, wobei:
die Steuereinrichtung bestimmte Information und bestimmte auswählbare Optionen zur Kontrolle über Funktionen des medizinischen Instruments auf der Anzeige darstellt; und
die Steuereinrichtung Information über gesperrte Unterprogramme und Funktionen auf der Anzeige nicht darstellt.

10. System zur Steuerung des Zugriffs nach Anspruch 7, wobei freigegebene Funktionen Gruppen von Betriebsfunktionen aufweisen, die sich auf bestimmte Umgangsorte in einer medizinischen Einrichtung beziehen.

11. System zur Steuerung des Zugriffs nach Anspruch 1, wobei die Steuereinrichtung die Authentizität des Zugriffsschlüssels durch Anwendung einer Integritätsprüfung überprüft.

12. Verfahren zur Steuerung des Zugriffs auf Betriebsfunktionen eines medizinischen Instruments, das eine Vielzahl von Betriebsfunktionen hat, wobei das Verfahren umfasst:
Bereitstellen von Steuersignalen für den Betrieb des medizinischen Instruments, wobei die Steuersignale Auswahlsignale aufweisen, um einzelne Funktionen oder Kombinationen von Funktionen für den Betrieb des medizinischen Instruments auszuwählen;
Bereitstellen eines Zugriffssignals, wobei das Zugriffssignal eine Zugriffskomponente und eine Funktionssteuerungskomponente hat, wobei die Funktionssteuerungskomponente einen Datenbereich aufweist, der angibt, welche der Betriebsfunktionen zu aktivieren oder zu deaktivieren sind;
Bestimmen, ob die Zugriffskomponente annehmbar ist, und wenn ja, Freigeben und Sperren bestimmter Betriebsfunktionen des medizinischen Instruments entsprechend dem Datenbereich der Funktionssteuerungskomponente des Zugriffsschlüssels; und
Annehmen von Steuersignalen zur Bedienerkontrolle über solche freigegebenen Funktionen und Nichtannehmen der Bedienerkontrolle über solche gesperrten Funktionen.

13. Verfahren zur Steuerung des Zugriffs nach Anspruch 12, wobei:
das Bereitstellen eines Zugriffssignals einschließt: Bereitstellen einer Funktionssteuerungskomponente, die ein Funktionspaket aufweist, in dem eine Vielzahl von Betriebsfunktionen in einem Funktionspaket enthalten sind; und
wenn das Funktionspaket freigegeben ist, Annehmen von Steuersignalen zur Bedienerkontrolle über alle Funktionen, die in dem Funktionspaket enthalten sind.

14. Verfahren zur Steuerung des Zugriffs nach Anspruch 12, wobei das Bereitstellen eines Zugriffssignals einschließt: Bereitstellen einer Versionsfreigabenummer.

15. Verfahren zur Steuerung des Zugriffs nach Anspruch 12, wobei das Bereitstellen eines Zugriffssignals einschließt: Bereitstellen einer Programmversion.

16. Verfahren zur Steuerung des Zugriffs nach Anspruch 12, ferner umfassend den folgenden Schritt:
Ansprechen auf Bedienersteuersignale für eine Basisbetriebsfunktion ungeachtet der Bereitstellung eines Zugriffssignals.

17. Verfahren zur Steuerung des Zugriffs nach Anspruch 12, ferner umfassend:
Installieren eines Steuerungsprogramms, das das medizinische Instrument steuert, wobei das Steuerungsprogramm eine Vielzahl von Unterprogrammen aufweist, die die Betriebsfunktionen steuern; und
Freigeben bestimmter Unterprogramme und Sperren anderer Unterprogramme durch das Steuerungsprogramm als Antwort auf die Funktionskomponente des Zugriffsschlüssels.

18. Verfahren zur Steuerung des Zugriffs nach Anspruch 17, ferner umfassend: Installieren des Steuerungsprogramms mit der Vielzahl von Unterprogrammen in einem Speicher, der sich innerhalb des medizinischen Instruments befindet, wobei die Vielzahl von Unterprogrammen alle Betriebsfunktionen des medizinischen Instruments steuert, über die ein Bediener die Kontrolle ausüben kann, wenn das jeweilige Unterprogramm freigegeben ist; und
wobei die Schritte des Freigebens bestimmter Unterprogramme und des Sperrens anderer Unterprogramme **dadurch** darüber bestimmen, welche Funktionen des medizinischen Instruments zur Verwendung durch einen Bediener verfügbar sind.

19. Verfahren zur Steuerung des Zugriffs nach Anspruch 18, ferner umfassend: Anzeigen bestimmter Information und bestimmter auswählbarer Optionen zur Kontrolle über Funktionen des medizinischen Instruments; und
Sperren einer Anzeige von Information über gesperrte Unterprogramme und Funktionen auf der Anzeige.

20. Verfahren zur Steuerung des Zugriffs nach Anspruch 12, ferner umfassend: Überprüfen der Authentizität des Zugriffssignals durch Anwendung einer Integritätsprüfung.

## Revendications

1. Système de contrôle d'accès à des fonctions opérationnelles d'un instrument médical qui a une pluralité de fonctions opérationnelles, le système comprenant :
un dispositif d'entrée par lequel un opérateur peut fournir des signaux de commande pour la mise en oeuvre de l'instrument médical, les signaux de commande comprenant des signaux de sélection pour sélectionner des fonctions uniques ou des combinaisons de fonctions pour la mise en oeuvre du composant médical, une clé d'accès ayant une composante d'accès et une composante de commande de fonction, dans lequel la composante de commande de fonction comprend une zone de données indiquant lesquelles des fonctions opérationnelles doivent être activées ou désactivées ; et
un contrôleur sensible à la clé d'accès pour déterminer si la composante d'accès est acceptable et, si c'est le cas, pour activer et désactiver des fonctions opérationnelles particulières de l'instrument médical conformément à la zone de données de la composante de commande de fonction de la clé d'accès, dans lequel le contrôleur est sensible aux signaux de commande provenant du dispositif d'entrée pour permettre le contrôle de l'opérateur sur ces fonctions activées et ne pas permettre le contrôle de l'opérateur sur ces fonctions désactivées.

2. Système de contrôle d'accès selon la revendication 1, dans lequel le contrôleur est sensible à des ensembles de fonctions, dans lequel une pluralité de fonctions sont incluses dans un ensemble de fonctions et, si elles sont activées par la clé d'accès, permettent à l'opérateur de l'instrument médical de sélectionner des ensembles de fonctions activées pour, de ce fait, appliquer toutes les fonctions de l'ensemble à l'instrument médical.

3. Système de contrôle d'accès selon la revendication 1, dans lequel la composante d'accès comprend une identification d'un numéro de mise en circulation.

4. Système de contrôle d'accès selon la revendication 3, dans lequel la composante d'accès comprend également une identification d'une version de programme.

5. Système de contrôle d'accès selon la revendication 3, dans lequel le contrôleur est sensible à des commandes d'opérateur provenant du dispositif d'entrée pour une fonction de fonctionnement de base indépendamment de la clé d'accès.

6. Système de contrôle d'accès selon la revendication 1, dans lequel le contrôleur et le dispositif d'entrée font partie de l'instrument médical.

7. Système de contrôle d'accès selon la revendication 1, dans lequel :
le contrôleur comprend un programme qui commande l'instrument médical, le programme comprenant une pluralité de sous-programmes qui commandent les fonctions opérationnelles ; et
le contrôleur est sensible à la composante de fonction de la clé d'accès pour activer certains sous-programmes et désactiver d'autres sous-programmes.

8. Système de contrôle d'accès selon la revendication 7, comprenant en outre une mémoire située dans l'instrument médical, dans lequel :
le contrôleur est situé dans l'instrument médical et est en communication avec la mémoire ;
un programme de commande d'instrument médical est mémorisé dans la mémoire, le programme de commande comportant une pluralité de sous-programmes qui commandent toutes les fonctions opérationnelles de l'instrument médical sur lesquelles un opérateur pourrait exercer un contrôle si le sous-programme respectif est activé ; et
le contrôleur est sensible à la composante de fonction de la clé d'accès pour activer certains sous-programmes et désactiver d'autres sous-programmes, commandant de ce fait les fonctions de l'instrument médical qui sont disponibles pour une utilisation par un opérateur.

9. Système de contrôle d'accès selon la revendication 8, comprenant en outre un afficheur, dans lequel :
le contrôleur présente certaines informations et certaines options sélectionnables pour la commande de fonctions de l'instrument médical sur l'afficheur ; et
le contrôleur ne présente pas d'informations concernant les sous-programmes et les fonctions désactivés sur l'afficheur.

10. Système de contrôle d'accès selon la revendication 7, dans lequel les fonctions activées comprennent des groupes de fonctions opérationnelles liés à des emplacements particuliers d'utilisation dans une structure médicale.

11. Système de contrôle d'accès selon la revendication 1, dans lequel le contrôleur vérifie l'authenticité de la clé d'accès en appliquant un contrôle d'intégrité.

12. Procédé de contrôle d'accès à des fonctions opérationnelles d'un instrument médical qui a une pluralité de fonctions opérationnelles, le procédé consistant à :
fournir des signaux de commande pour la mise en oeuvre de l'instrument médical, les signaux de commande comprenant des signaux de sélection pour sélectionner des fonctions uniques ou des combinaisons de fonctions pour la mise en oeuvre de l'instrument médical ;
fournir un signal d'accès, le signal d'accès comportant une composante d'accès et une composante de commande de fonction, dans lequel la composante de commande de fonction comprend une zone de données indiquant lesquelles des fonctions opérationnelles doivent être activées ou désactivées ;
déterminer si la composante d'accès est acceptable et, si c'est le cas, activer et désactiver des fonctions opérationnelles particulières de l'instrument médical conformément à la zone de données de la composante de commande de fonction de la clé d'accès ; et
accepter des signaux de commande pour le contrôle de l'opérateur sur ces fonctions activées et ne pas accepter le contrôle de l'opérateur sur ces fonctions désactivées.

13. Procédé de contrôle d'accès selon la revendication 12, dans lequel :
la fourniture d'un signal d'accès comprend la fourniture d'une composante de commande de fonction qui comprend un ensemble de fonctions, dans lequel une pluralité de fonctions opérationnelles sont incluses dans un ensemble de fonctions ; et
lorsque l'ensemble de fonctions est activé, accepter des signaux de commande pour le contrôle de l'opérateur sur toutes les fonctions incluses dans l'ensemble de fonctions.

14. Procédé de contrôle d'accès selon la revendication 12, dans lequel la fourniture d'un signal d'accès comprend la fourniture d'un numéro de mise en circulation.

15. Procédé de contrôle d'accès selon la revendication 12, dans lequel la fourniture d'un signal d'accès comprend la fourniture d'une version de programme.

16. Procédé de contrôle d'accès selon la revendication 12, comprenant en outre l'étape consistant à répondre à des signaux de commande d'opérateur pour une fonction de fonctionnement de base indépendamment de la fourniture d'un signal d'accès.

17. Procédé de contrôle d'accès selon la revendication 12, consistant en outre à :
installer un programme de commande qui commande l'instrument médical, le programme de commande comprenant une pluralité de sous-programmes qui commandent les fonctions opérationnelles ; et
activer certains sous-programmes et désactiver d'autres sous-programmes par le programme de commande en réponse à la composante de fonction de la clé d'accès.

18. Procédé de contrôle d'accès selon la revendication 17, comprenant en outre l'installation du programme de commande comportant la pluralité de sous-programmes dans une mémoire située dans l'instrument médical, la pluralité de sous-programmes commandant toutes les fonctions opérationnelles de l'instrument médical sur lesquelles un opérateur pourrait exercer un contrôle si le sous-programme respectif est activé ; et
les étapes consistant à activer certains sous-programmes et désactiver d'autres sous-programmes, commandant de ce fait les fonctions de l'instrument qui sont disponibles pour une utilisation par un opérateur.

19. Procédé de contrôle d'accès selon la revendication 18, comprenant en outre l'affichage de certaines informations et de certaines options sélectionnables pour la commande de fonctions de l'instrument médical ; et
la désactivation d'un affichage d'informations concernant des sous-programmes et des fonctions désactivés sur l'afficheur.

20. Procédé de contrôle d'accès selon la revendication 12, comprenant en outre la vérification de l'authenticité du signal d'accès en appliquant un contrôle d'intégrité.
